# EUROPEAN PATENT APPLICATION

(11) **EP 4 691 453 A1**
(43) Date of publication of application: **11.02.2026**
(21) Application number: 24779470.4
(22) Date of filing: 13.03.2024
(51) Int. Cl.: A61K 8/37, A61K 8/06, A61K 8/27, A61K 8/92, A61Q 17/04, A61Q 19/00

(54) **COSMETIC COMPOSITION**

(30) Priority: 27.03.2023 JP 2023050332
(71) Applicant: Shiseido Company, Ltd., Chuo-ku Tokyo 104-0061 (JP)
(72) Inventor: HIGUCHI, Tomoki, Tokyo 104-0061 (JP)
(74) Representative: Ter Meer Steinmeister & Partner
(86) International application number: PCT/JP2024/009801
(87) International publication number: WO 2024/203341

(57) **Abstract**

Provided is a cosmetic composition in which Compound I having a structure of formula (I) below is contained and solubility of Compound I in oil is maintained or improved.

A cosmetic composition, comprising
Compound I having a structure of formula (I) below: 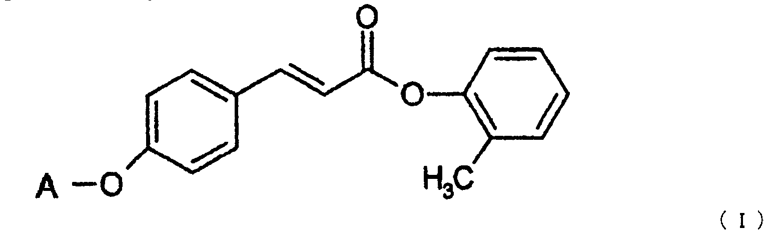 wherein -OA represents an alkoxy group;
a polar oil;
a first silicone oil having a kinematic viscosity at 25°C of 10 cSt or more and lacking a benzene ring; and
a compatibilizing oil compatible with both the polar oil and the first silicone oil.

## Description

### FIELD

The present invention relates to a cosmetic composition.

### BACKGROUND

Ultraviolet rays are said to cause oxidation of sebum and damage to cellular DNA by generating free radicals *in vivo.* The adverse effects on the skin due to such effect of ultraviolet rays include negative effects such as skin cancer, photoaging, spots, wrinkles, and inflammation, and are not preferable from the viewpoints of health and beauty.

Examples of the types of ultraviolet rays include medium-wavelength ultraviolet rays (UVB region: wavelengths of 290 to 320 nm) that are known to cause sunburn and inflammation and long-wavelength ultraviolet rays (UVA region: wavelengths of 320 to 400 nm) that are known to cause photoaging.

Numerous ultraviolet absorbers for protecting the skin from ultraviolet rays have been developed to date. For example, the ultraviolet absorber disclosed in PTL 1 has an absorption capacity in both the UVA region and the UVB region, has an ultraviolet-suppressing effect in a wide range of wavelengths, and has a characteristic not seen in the prior art, where the ultraviolet absorption capacity in the UVA and UVB regions increases with the passage of time during ultraviolet irradiation.

More specifically, the ultraviolet absorber of PTL 1 comprises, as an active component, a compound represented by general formula I: 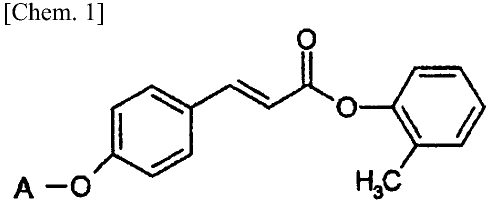 wherein -OA represents an alkoxy group.

### [CITATION LIST]

### [PATENT LITERATURE]

[PTL 1] WO 2009/041098

### SUMMARY

### [TECHNICAL PROBLEM]

The compound of the general formula I in PTL 1 above is known to have a physical property of dissolving in a polar oil.

However, when a polar oil is blended in a cosmetic in a large amount, there is a possibility that usability of the cosmetic may be limited.

In addition to polar oils, hydrocarbon oils and short-chain silicone oils having satisfactory compatibility therewith are often blended in cosmetics. However, through intensive research by the present inventors, it was found that when a hydrocarbon oil and a short-chain silicone oil, in addition to a polar oil, are blended in a cosmetic as oils, solubility of the above compound of the general formula I in oil may be reduced.

The present invention aims to improve the above circumstances, and an object thereof is to provide a cosmetic composition in which Compound I having a structure of formula (I) (identical to the above general formula I) is contained and solubility of Compound I in oil is maintained or improved.

### [SOLUTION TO PROBLEM]

The present invention that achieves the above object is as follows.

### <Aspect 1>

A cosmetic composition, comprising
Compound I having a structure of formula (I) below: 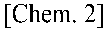 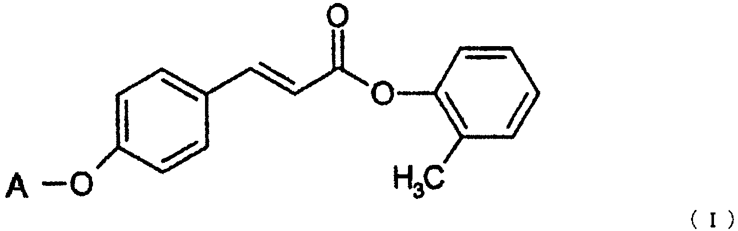
wherein -OA represents an alkoxy group;
a polar oil;
a first silicone oil having a kinematic viscosity at 25°C of 10 cSt or more and lacking a benzene ring; and
a compatibilizing oil compatible with both the polar oil and the first silicone oil.

### <Aspect 2>

The cosmetic composition according to Aspect 1, wherein the compatibilizing oil is a hydrocarbon oil and/or a second silicone oil having a kinematic viscosity at 25°C of 6 cSt or less.

### <Aspect 3>

The cosmetic composition according to Aspect 1 or 2, wherein the Compound I is 0.1 to 10 parts by mass relative to 100 parts by mass in total of the Compound I, the polar oil, the first silicone oil, and the compatibilizing oil.

### <Aspect 4>

The cosmetic composition according to any one of Aspects 1 to 3, wherein the polar oil is 0.1 to 99 parts by mass relative to 100 parts by mass in total of the Compound I and the polar oil.

### <Aspect 5>

The cosmetic composition according to any one of Aspects 1 to 4, wherein the first silicone oil is 0.1 to 20 parts by mass relative to 100 parts by mass in total of the Compound I, the polar oil, the first silicone oil, and the compatibilizing oil.

### <Aspect 6>

The cosmetic composition according to any one of Aspects 1 to 5, wherein the compatibilizing oil is 10 to 90 parts by mass relative to 100 parts by mass in total of the first silicone oil and the compatibilizing oil.

### <Aspect 7>

An oil-in-water emulsion cosmetic, comprising the cosmetic composition according to any one of Aspects 1 to 6.

### <Aspect 8>

The oil-in-water emulsion cosmetic according to Aspect 7, wherein content of the Compound I is 0.01 to 10% by mass.

### <Aspect 9>

The oil-in-water emulsion cosmetic according to Aspect 7 or 8, wherein content of the polar oil is 0.1 to 50% by mass.

### <Aspect 10>

A water-in-oil emulsion cosmetic, comprising the cosmetic composition according to any one of Aspects 1 to 6.

### <Aspect 11>

The water-in-oil emulsion cosmetic according to Aspect 10, wherein content of the Compound I is 0.01 to 10% by mass.

### <Aspect 12>

The water-in-oil emulsion cosmetic according to Aspect 10 or 11, wherein content of the polar oil is 0.1 to 50% by mass.

### [ADVANTAGEOUS EFFECTS OF INVENTION]

According to the present invention, a cosmetic composition in which Compound I having a structure of formula I is contained and solubility of Compound I in oil is maintained or improved can be provided.

### BRIEF DESCRIPTION OF DRAWINGS

FIG. 1 is a chart showing absorbance of each of the samples of Examples 1 and 2, Comparative Example 1, and Reference Examples 1 and 2.
FIG. 2 is a chart showing absorbance of each of the samples of Examples 3 and 4, Comparative Examples 2 and 3, and Reference Examples 3 and 4.

### DESCRIPTION OF EMBODIMENTS

Hereinafter, embodiments of the present invention will be described in detail. The present invention is not limited to the following embodiments, and various modifications can be made within the scope of the invention.

### <<Cosmetic composition>>

The cosmetic composition of the present invention (hereinafter, also simply referred to as "composition of the present invention") is
a cosmetic composition, comprising or consisting of
Compound I having a structure of formula (I) below: 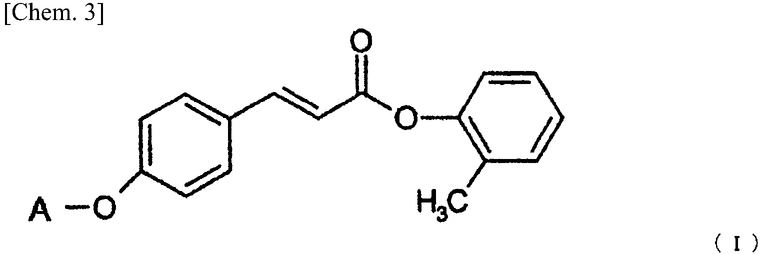
wherein -OA represents an alkoxy group;
a polar oil;
a first silicone oil having a kinematic viscosity at 25°C of 10 cSt or more and lacking a benzene ring; and
a compatibilizing oil compatible with both the polar oil and the first silicone oil.

As described above, it was found that when a hydrocarbon oil and a short-chain silicone oil, in addition to a polar oil, are used as oils, solubility of Compound I in oil may be reduced.

According to further intensive research by the present inventors, it was discovered that by blending in a polar oil a silicone oil (i.e., the above "first silicone oil") having a specific kinematic viscosity and having a specific structure and a compatibilizing oil compatible with both the polar oil and the first silicone oil, solubility of Compound I can be maintained or improved.

Generally, in a cosmetic composition in which a polar oil is blended, a hydrocarbon oil and/or a short-chain silicone oil (for example, a silicone oil having a kinematic viscosity at 25°C of less than 10 cSt) are often used in combination as an additional oil component. The reason therefor is that hydrocarbon oils and short-chain silicone oils have satisfactory compatibility with polar oils, and therefore when these are mixed, a mutually compatible state is obtained.

In the present invention, surprisingly, by intentionally selecting a "first silicone oil" that is generally incompatible or poorly compatible with a polar oil and blending a compatibilizing oil compatible with both the polar oil and the first silicone oil, solubility of Compound I can be maintained or improved without increasing the content of the polar oil.

In the following, each component that can be contained in the composition of the present invention will be described in detail.

### <Compound I>

Compound I contained in the composition of the present invention has a structure of formula (I) below: 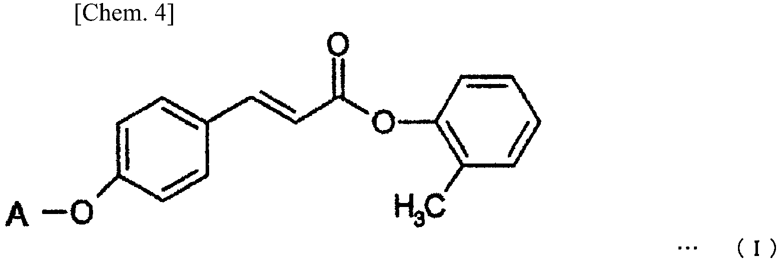 wherein -OA represents an alkoxy group.

In the formula (I), -OA represents an alkoxy group, and more specifically, includes, for example, a methoxy group and an ethoxy group, but is not limited thereto. This Compound I can be used as a UVA and UVB absorber.

In the present invention, Compound I may be identical in structure as the compound represented by the general formula I disclosed in PTL 1. In the present invention, Compound I, for example, may be 2-methylphenyl 4-methoxycinnamate.

In the composition of the present invention, the content of Compound I is not particularly limited and, for example, the content of Compound I may be adjusted as follows.

In the composition of the present invention, Compound I, for example, may be 0.1 parts by mass or more, 0.5 parts by mass or more, 1.0 parts by mass or more, 1.5 parts by mass or more, 2.0 parts by mass or more, 2.5 parts by mass or more, 3.0 parts by mass or more, 3.5 parts by mass or more, 4.0 parts by mass or more, 4.5 parts by mass or more, 5.0 parts by mass or more, 5.5 parts by mass or more, 6.0 parts by mass or more, 6.5 parts by mass or more, 7.0 parts by mass or more, 7.5 parts by mass or more, 8.0 parts by mass or more, 8.5 parts by mass or more, 9.0 parts by mass or more, 9.5 parts by mass or more, or 10 parts by mass or more, and may be 15 parts by mass or less, 14 parts by mass or less, 13 parts by mass or less, 12 parts by mass or less, 11 parts by mass or less, or 10 parts by mass or less, relative to 100 parts by mass in total of Compound I, the polar oil, the first silicone oil, and the compatibilizing oil.

### <Polar oil>

In the present invention, the "polar oil" refers to a highly polar oil, among oils that can be used in cosmetics, other than a silicone oil, and refers to one having, for example, an IOB value of 0.10 or greater, 0.11 or greater, 0.12 or greater, or 0.13 or greater. In addition, the IOB value of the polar oil according to the present invention may be 1.50 or less, 1.00 or less, 0.95 or less, 0.90 or less, 0.85 or less, 0.80 or less, 0.75 or less, 0.70 or less, 0.65 or less, 0.60 or less, 0.55 or less, 0.50 or less, 0.45 or less, or 0.40 or less.

IOB value is an abbreviation for inorganic/organic balance, is a value that represents a ratio of inorganic value to organic value, and acts as an indicator showing the degree of polarity of an organic compound. The IOB value, specifically, is represented as IOB value = inorganic value / organic value. With respect to each of "inorganic value" and "organic value", the "inorganic value" and "organic value" are set according to various atoms or functional groups, such that, for example, the "organic value" for one carbon atom in a molecule is 20, and the "inorganic value" for one hydroxyl group is 100, and by summing the "inorganic values" and "organic values" of all atoms and functional groups in an organic compound, the IOB value of the organic compound can be calculated (refer to, for example, Yoshio Koda, "Organic Conceptual Diagrams - Fundamentals and Applications -", pp. 11 to 17, Sankyo Publishing Co., Ltd., published in 1984).

In the present invention, the polar oil, for example, may be at least one selected from the group consisting of ester oils, ether oils, higher alcohols, and fatty acids, having an IOB value of 0.10 or greater.

More specifically, the polar oil, for example, may be at least one selected from the group consisting of bis-ethoxydiglycol cyclohexane-1,4-dicarboxylate (IOB value = 1.03), phenoxyethyl caprylate (IOB value = 0.29), PPG-30-buteth-30 (IOB value = 0.94), diisopropyl sebacate (IOB value =0.4), neopentyl glycol diheptanoate (IOB value = 0.33), diethoxyethyl succinate (IOB value = 1.13), diethylhexyl succinate (IOB value = 0.32), isononyl isononanoate (IOB value = 0.2), isopropyl myristate (IOB value = 0.18), octyl palmitate (IOB value = 0.13), isopropyl palmitate (IOB value = 0.16), butyl stearate (IOB value = 0.14), hexyl laurate (IOB value = 0.17), myristyl myristate (IOB value = 0.11), decyl oleate (IOB value = 0.11), isotridecyl isononanoate (IOB value = 0.15), cetyl ethylhexanoate (IOB value = 0.13), pentaerythrityl tetraethylhexanoate (IOB value = 0.35), dioctyl succinate (IOB value = 0.36), glycol distearate (IOB value = 0.16), glyceryl diisostearate (IOB value = 0.29), neopentyl glycol dicaprate (IOB value = 0.25), diisostearyl malate (IOB value = 0.28), trimethylolpropane triisostearate (IOB value = 0.16), glyceryl tri-2-ethylhexanoate (triethylhexanoin) (IOB value = 0.35), trimethylolpropane trioctanoate (IOB value = 0.33), trimethylolpropane triisostearate (IOB value = 0.16), diisobutyl adipate (IOB value = 0.46), N-lauroyl-L-glutamic acid-2-octyldodecyl ester (IOB value = 0.29), 2-hexyldecyl adipate (IOB value = 0.16), ethylhexyl methoxycinnamate (IOB value = 0.28), octocrylene (IOB value = 0.32), ethylhexyl salicylate (IOB value = 0.58), alkyl benzoate (C12-15) (IOB value = 0.17 to 0.2), phenethyl benzoate (IOB value = 0.30), butyloctyl salicylate (IOB value = 0.46), 2-ethylhexyl palmitate (IOB value = 0.13), 2-ethylhexyl ethylhexanoate (IOB value = 0.2), triisostearin (IOB value = 0.16), PPG-3 dipivalate (IOB value = 0.52), and glyceryl tri(caprylate/caprate) (IOB value = 0.33), but are not limited thereto.

In the composition of the present invention, the content of the polar oil is not particularly limited and, for example, the content of the polar oil may be adjusted as follows.

In the composition of the present invention, the polar oil, for example, may be 0.1 parts by mass or more, 1.0 parts by mass or more, 2.0 parts by mass or more, 3.0 parts by mass or more, 5.0 parts by mass or more, 5.0 parts by mass or more, 6.0 parts by mass or more, 7.0 parts by mass or more, 8.0 parts by mass or more, 9.0 parts by mass or more, 10 parts by mass or more, 15 parts by mass or more, 20 parts by mass or more, 25 parts by mass or more, 30 parts by mass or more, 35 parts by mass or more, 40 parts by mass or more, 45 parts by mass or more, 50 parts by mass or more, 55 parts by mass or more, 60 parts by mass or more, 65 parts by mass or more, 70 parts by mass or more, 75 parts by mass or more, 80 parts by mass or more, or 85 parts by mass or more, and may be 99 parts by mass or less, 95 parts by mass or less, 90 parts by mass or less, 85 parts by mass or less, 80 parts by mass or less, 75 parts by mass or less, 70 parts by mass or less, 65 parts by mass or less, 60 parts by mass or less, 55 parts by mass or less, 50 parts by mass or less, 45 parts by mass or less, 40 parts by mass or less, 35 parts by mass or less, 30 parts by mass or less, 25 parts by mass or less, 20 parts by mass or less, 15 parts by mass or less, 10 parts by mass or less, or 8.0 parts by mass or less, relative to 100 parts by mass in total of Compound I and the polar oil.

In the composition of the present invention, the content of the polar oil may be adjusted in accordance with the content of the first silicone oil and the content of Compound I used in combination therewith.

### <First silicone oil>

The first silicone oil contained in the composition of the present invention has a kinematic viscosity at 25°C of 10 cSt or more and lacks a benzene ring.

In the present invention, "kinematic viscosity" is the absolute viscosity of a liquid oil divided by the density thereof, and can be measured based on the principle that the time for a liquid oil to descend through a capillary tube is proportional to the viscosity thereof. In the present invention, kinematic viscosities of the first silicone oil and the second silicone oil described below are measured at 25°C, and are in units of cSt (centistokes).

It is known that kinematic viscosity of a silicone oil is generally proportional to the length of the main backbone formed by siloxane bonds in the silicone oil. More specifically, it is known that the shorter the main backbone of a silicone oil, the lower the kinematic viscosity thereof, and conversely, the longer the main backbone of a silicone oil, the higher the kinematic viscosity thereof.

As described above, short-chain silicone oils (i.e., silicone oils having a relatively low kinematic viscosity, more specifically, for example, silicone oils having a kinematic viscosity at 25°C of less than 10 cSt) have satisfactory compatibility with polar oils, and therefore are often used in combination with polar oils. The composition of the present invention is characterized in that a first silicone oil that is generally incompatible or poorly compatible with a polar oil and has a kinematic viscosity at 25°C of 10 cSt or more is intentionally used.

In the present invention, the kinematic viscosity at 25°C of the first silicone oil, more specifically, for example, may be 10 cSt or more, 15 cSt or more, 20 cSt or more, 25 cSt or more, 30 cSt or more, 35 cSt or more, 40 cSt or more, 45 cSt or more, 50 cSt or more, 55 cSt or more, 60 cSt or more, 65 cSt or more, 70 cSt or more, 75 cSt or more, 80 cSt or more, 85 cSt or more, 90 cSt or more, 95 cSt or more, or 100 cSt or more, and may be 1000 cSt or less, 800 cSt or less, 500 cSt or less, 100 cSt or less, 90 cSt or less, 80 cSt or less, 70 cSt or less, 60 cSt or less, 50 cSt or less, 40 cSt or less, 30 cSt or less, or 20 cSt or less.

Silicone oils having a benzene ring in the structure thereof (for examples, Cases 6 and 7 described below) are generally highly polar and exhibit satisfactory compatibility with polar oils, and therefore there is low inventiveness from the viewpoint of dissolving Compound I. The composition of the present invention is characterized in that a first silicone oil lacking a benzene ring in the structure thereof is used.

In the present invention, the first silicone oil lacking a benzene ring is not particularly limited and, for example, may be a linear silicone oil lacking a benzene ring.

More specifically, the first silicone oil according to the present invention, for example, may be dimethicone ("methyl polysiloxane").

In the present invention, a commercially available product may be used as the first silicone oil, for example, silicone oils manufactured by Shin-Etsu Chemical Co., Ltd. under trade names such as "KF-96A-10CS", "KF-96A-20CS", "KF-96A-30CS", "KF-96A-50CS", "KF-96A-100CS", and "KF-96A-200CS" may be used, but is not limited thereto.

In the present invention, the first silicone oil may be non-volatile.

In the present invention, an oil being "volatile" means an oil having a boiling point of 260°C or lower at normal pressure (1 atmosphere). In addition, in the present invention, an oil being "non-volatile" means an oil having a boiling point higher than 260°C under atmospheric pressure or normal pressure (1 atmosphere).

In the composition of the present invention, the content of the first silicone oil is not particularly limited and, for example, the content of the first silicone oil may be adjusted as follows.

In the composition of the present invention, the first silicone oil, for example, may be 0.1 parts by mass or more, 0.5 parts by mass or more, 1.0 parts by mass or more, 1.5 parts by mass or more, 2.0 parts by mass or more, 2.5 parts by mass or more, 3.0 parts by mass or more, 3.5 parts by mass or more, 4.0 parts by mass or more, 4.5 parts by mass or more, 5.0 parts by mass or more, 5.5 parts by mass or more, 6.0 parts by mass or more, 6.5 parts by mass or more, 7.0 parts by mass or more, 7.5 parts by mass or more, 8.0 parts by mass or more, 8.5 parts by mass or more, 9.0 parts by mass or more, or 10 parts by mass or more, and may be 20 parts by mass or less, 19 parts by mass or less, 18 parts by mass or less, 17 parts by mass or less, 16 parts by mass or less, 15 parts by mass or less, 14 parts by mass or less, 13 parts by mass or less, 12 parts by mass or less, 11 parts by mass or less, 10 parts by mass or less, or 9.0 parts by mass or less, relative to 100 parts by mass in total of Compound I, the polar oil, the first silicone oil, and the compatibilizing oil.

### <Compatibilizing oil>

The compatibilizing oil contained in the composition of the present invention is an oil compatible with both the polar oil and the first silicone oil described above. Including the compatibilizing oil in the composition of the present invention allows the polar oil and the first silicone oil to be compatibilized. In other words, in the composition of the present invention, the polar oil, the first silicone oil, and the compatibilizing oil can be mutually dissolved into a uniformly mixed state by stirring as needed, i.e., can be visually observed as a monolayer oil phase.

In the composition of the present invention, the content of the compatibilizing oil is not particularly limited and, for example, the content of the compatibilizing oil may be adjusted as follows.

In the composition of the present invention, the compatibilizing oil, for example, may be 10 parts by mass or more, 20 parts by mass or more, 30 parts by mass or more, 40 parts by mass or more, or 50 parts by mass or more, and may be 90 parts by mass or less, 80 parts by mass or less, 70 parts by mass or less, 60 parts by mass or less, or 50 parts by mass or less, relative to 100 parts by mass in total of the first silicone oil and the compatibilizing oil.

The compatibilizing oil contained in the composition of the present invention needs only to be an oil compatible with both the polar oil and the first silicone oil described above, and is not particularly limited. More specifically, in the composition of the present invention, the compatibilizing oil, for example, may be a hydrocarbon oil and/or a second silicone oil having a kinematic viscosity at 25°C of 6 cSt or less.

### (Hydrocarbon oil)

In the present invention, the hydrocarbon oil refers to a hydrocarbon oil other than the polar oil described above.

The hydrocarbon oil may be a volatile hydrocarbon oil, or may be a non-volatile hydrocarbon oil.

Specific examples of the hydrocarbon oil include decane, dodecane, isododecane, isohexadecane, liquid paraffin, squalane, squalene, paraffin, and mixtures of two or more thereof, but are not limited thereto.

### (Second silicone oil)

In the present invention, the second silicone oil refers to a silicone oil having a kinematic viscosity at 25°C of 6 cSt or less.

More specifically, the kinematic viscosity at 25°C of the second silicone oil, for example, may be 6 cSt or less, 5.5 cSt or less, 5.0 cSt or less, 4.5 cSt or less, 4.0 cSt or less, 3.5 cSt or less, 3.0 cSt or less, 2.5 cSt or less, 2.0 cSt or less, or 1.5 cSt or less, and may be 1.0 cSt or more or 1.5 cSt or more.

More specifically, the second silicone oil according to the present invention, for example, may be dimethicone ("methyl polysiloxane").

In the present invention, a commercially available product may be used as the second silicone oil, for example, silicone oils manufactured by Shin-Etsu Chemical Co., Ltd. under trade names such as "KF-96L-1.5CS", "KF-96A-6T", "KF-96L-1CS", and "KF-96L-2CS" may be used, but is not limited thereto.

In the present invention, the second silicone oil may be volatile or may be non-volatile, but preferably comprises a volatile one.

### <Additional component>

The cosmetic composition of the present invention may further comprise any aqueous component or additional component that can be used in the cosmetic field, other than the components described above. The additional component, for example, may further include additional components such as oils other than the oils described above, ultraviolet-scattering particles, ultraviolet absorbers, surfactants, moisturizers, sequestering agents, natural and synthetic polymers, water-soluble and oil-soluble polymers, various extracts, coloring agents such as organic dyes, preservatives, antioxidants, pigments, thickeners, pH adjusters, fragrances, cooling agents, antiperspirants, bactericides, skin activators, other pharmaceuticals, and various powders, but is not limited thereto.

### <<Cosmetic>>

The composition of the present invention described above can be suitably used as a cosmetic or a raw material thereof. In addition, the composition of the present invention, for example, can be suitably used as a sunscreen cosmetic, a cosmetic primer, a daytime emulsion, or a raw material thereof.

The dosage form of the cosmetic of the present invention is not particularly limited, and examples include liquid, emulsion, gel, and cream, but is not limited thereto.

The present invention, for example, can provide an oil-in-water emulsion cosmetic comprising the composition of the present invention, a water-in-oil emulsion cosmetic comprising the composition of the present invention, and an oily cosmetic comprising the composition of the present invention.

### <Oil-in-water emulsion cosmetic composition>

The oil-in-water emulsion cosmetic composition according to the present invention may include in a dispersed phase (internal phase) thereof the composition of the present invention.

In the oil-in-water emulsion cosmetic according to the present invention, the content of Compound I is not particularly limited and, for example, may be 0.01% by mass or greater, 0.05% by mass or greater, 0.1% by mass or greater, 0.5% by mass or greater, 1.0% by mass or greater, 1.5% by mass or greater, 2.0% by mass or greater, 2.5% by mass or greater, or 3.0% by mass or greater, and may be 10% by mass or less, 9.0% by mass or less, 8.0% by mass or less, 7.0% by mass or less, 6.0% by mass or less, 5.0% by mass or less, 4.0% by mass or less, or 3.0% by mass or less, relative to the total amount of the cosmetic.

In the oil-in-water emulsion cosmetic according to the present invention, the content of the polar oil is not particularly limited and, for example, may be 0.1% by mass or greater, 0.5% by mass or greater, 1.0% by mass or greater, 5.0% by mass or greater, 10% by mass or greater, 15% by mass or greater, or 20% by mass or greater, and may be 50% by mass or less, 45% by mass or less, 40% by mass or less, 35% by mass or less, 30% by mass or less, 25% by mass or less, or 20% by mass or less, relative to the total amount of the cosmetic.

In the oil-in-water emulsion cosmetic according to the present invention, the content of water is not particularly limited and, for example, may be 30% by mass or greater, 35% by mass or greater, 40% by mass or greater, 45% by mass or greater, 50% by mass or greater, 55% by mass or greater, 60% by mass or greater, 65% by mass or greater, 70% by mass or greater, 75% by mass or greater, 80% by mass or greater, or 85% by mass or greater, and may be 90% by mass or less, 85% by mass or less, 80% by mass or less, 75% by mass or less, 70% by mass or less, 65% by mass or less, 60% by mass or less, or 55% by mass or less, relative to the total amount of the cosmetic.

The oil-in-water emulsion cosmetic according to the present invention may further comprise a surfactant as needed. The content of the surfactant, if comprising any, is not particularly limited and, for example, may be 0.1% by mass or greater, 0.5% by mass or greater, or 1.0% by mass or greater, and may be 10% by mass or less, 5.0% by mass or less, or 3.0% by mass or less, relative to the total amount of the cosmetic.

The oil-in-water emulsion cosmetic according to the present invention may further comprise any aqueous component or additional component that can be used in the cosmetic field, other than the components described above. The additional component, for example, may further include additional components such as oils other than the oils described above, ultraviolet-scattering particles, ultraviolet absorbers, moisturizers, sequestering agents, natural and synthetic polymers, water-soluble and oil-soluble polymers, various extracts, coloring agents such as organic dyes, preservatives, antioxidants, pigments, thickeners, pH adjusters, fragrances, cooling agents, antiperspirants, bactericides, skin activators, other pharmaceuticals, and various powders, but is not limited thereto.

### <Water-in-oil emulsion cosmetic>

The water-in-oil emulsion cosmetic according to the present invention may include in a continuous phase (external phase) thereof the composition of the present invention.

In the water-in-oil emulsion cosmetic according to the present invention, the content of Compound I is not particularly limited and, for example, may be 0.01% by mass or greater, 0.05% by mass or greater, 0.1% by mass or greater, 0.5% by mass or greater, 1.0% by mass or greater, 1.5% by mass or greater, 2.0% by mass or greater, 2.5% by mass or greater, or 3.0% by mass or greater, and may be 10% by mass or less, 9.0% by mass or less, 8.0% by mass or less, 7.0% by mass or less, 6.0% by mass or less, 5.0% by mass or less, 4.0% by mass or less, or 3.0% by mass or less, relative to the total amount of the cosmetic.

In the water-in-oil emulsion cosmetic according to the present invention, the content of the polar oil is not particularly limited and, for example, may be 0.1% by mass or greater, 0.5% by mass or greater, 1.0% by mass or greater, 5.0% by mass or greater, 10% by mass or greater, 15% by mass or greater, or 20% by mass or greater, and may be 50% by mass or less, 45% by mass or less, 40% by mass or less, 35% by mass or less, 30% by mass or less, 25% by mass or less, or 20% by mass or less, relative to the total amount of the cosmetic.

In the water-in-oil emulsion cosmetic according to the present invention, the content of water is not particularly limited and, for example, may be 5.0% by mass or greater, 10% by mass or greater, 15% by mass or greater, 20% by mass or greater, 25% by mass or greater, 30% by mass or greater, 35% by mass or greater, 40% by mass or greater, 45% by mass or greater, or 50% by mass or greater, and may be 65% by mass or less, 60% by mass or less, or 55% by mass or less, relative to the total amount of the cosmetic.

The water-in-oil emulsion cosmetic according to the present invention may further comprise a surfactant as needed. The content of the surfactant, if comprising any, is not particularly limited and, for example, may be 0.1% by mass or greater, 0.5% by mass or greater, or 1.0% by mass or greater, and may be 10% by mass or less, 5.0% by mass or less, or 3.0% by mass or less, relative to the total amount of the cosmetic.

The water-in-oil emulsion cosmetic according to the present invention may further comprise any aqueous component or additional component that can be used in the cosmetic field, other than the components described above. The additional component, for example, may further include additional components such as oils other than the oils described above, ultraviolet-scattering particles, ultraviolet absorbers, moisturizers, sequestering agents, natural and synthetic polymers, water-soluble and oil-soluble polymers, various extracts, coloring agent such as organic dyes, preservatives, antioxidants, pigments, thickeners, pH adjusters, fragrances, cooling agents, antiperspirants, bactericides, skin activators, other pharmaceuticals, and various powders, but is not limited thereto.

### <Oily cosmetic>

In the present invention, an oily cosmetic refers to a cosmetic having an oil-phase monophasic system. In addition, the oily cosmetic generally does not substantially comprise water that is present separately from the oil phase.

In the oily cosmetic according to the present invention, the content of Compound I is not particularly limited and, for example, may be 0.01% by mass or greater, 0.05% by mass or greater, 0.1% by mass or greater, 0.5% by mass or greater, 1.0% by mass or greater, 1.5% by mass or greater, 2.0% by mass or greater, 2.5% by mass or greater, or 3.0% by mass or greater, and may be 10% by mass or less, 9.0% by mass or less, 8.0% by mass or less, 7.0% by mass or less, 6.0% by mass or less, 5.0% by mass or less, 4.0% by mass or less, or 3.0% by mass or less, relative to the total amount of the cosmetic.

In the oily cosmetic according to the present invention, the content of the polar oil is not particularly limited and, for example, may be 0.1% by mass or greater, 0.5% by mass or greater, 1.0% by mass or greater, 5.0% by mass or greater, 10% by mass or greater, 15% by mass or greater, or 20% by mass or greater, and may be 50% by mass or less, 45% by mass or less, 40% by mass or less, 35% by mass or less, 30% by mass or less, 25% by mass or less, or 20% by mass or less, relative to the total amount of the cosmetic.

The oily cosmetic according to the present invention may further comprise any aqueous component or additional component that can be used in the cosmetic field, other than the components described above. The additional component, for example, may further include additional components such as oils other than the oils described above, ultraviolet-scattering particles, ultraviolet absorbers, moisturizers, sequestering agents, natural and synthetic polymers, oil-soluble polymers, various extracts, coloring agents such as organic dyes, preservatives, antioxidants, pigments, thickeners, pH adjusters, fragrances, cooling agents, antiperspirants, bactericides, skin activators, other pharmaceuticals, and various powders, but is not limited thereto.

### EXAMPLES

The present invention will be further described in detail with reference to the Examples below. However, the present invention is not limited thereto. Hereinafter, unless specified otherwise, blending amounts are expressed as % by mass.

### <<Cases 1 to 7 Evaluation of solubility of Compound I in oil>>

Based on the composition in the following Table 1-1, the compositions of Cases 1 to 7 shown in Table 1-2 were prepared. The compound shown in the structural formula below was used (also used in the same manner in each of Cases 8 to 21 and each of the Examples and Comparative Examples shown below) as Compound I blended in each composition:

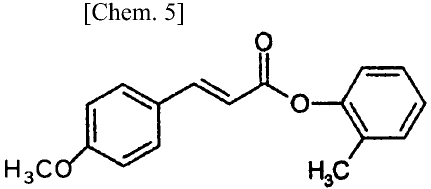

A total amount of 40 g of each composition was placed in a 50 mL screw-cap tube. Compound I was added in an amount equal to or greater than the solubility of Compound I with respect to the polar oil used.

Evaluation method: Heating to 80°C, Compound I was dissolved in the oil, and then left to stand in a thermostatic bath at 0°C for one week. The amount of crystal precipitation of Compound I was visually evaluated in three levels according to the following criteria. Evaluation results are shown in Table 1-2.

### (Evaluation criteria)

"A": No crystals were found;
"B": A small amount of crystals were observed;
"C": A large amount of crystals were observed.

**[Table 1-1]**

| (Table 1-1 Composition) | | | | |
|---|---|---|---|---|
| | Composition 1 | Composition 2 | Composition 3 | Composition 4 |
| Compound I | 2 | 2 | 2 | 2 |
| Isododecane (compatibilizing oil) | 38 | 27 | 11 | - |
| Diisopropyl sebacate (polar oil) | 60 | 60 | 60 | 60 |
| Silicone oil | - | 11 | 27 | 38 |
| Total | 100 | 100 | 100 | 100 |

| | | | | |
|---|---|---|---|---|
| *Numerical values in table represent blending amounts. | | | | |

**[Table 1-2]**

| (Table 1-2 Evaluation of solubility of Compound I) | | | | | | | |
|---|---|---|---|---|---|---|---|
| | Silicone oil | | | Composition 1 | Composition 2 | Composition 3 | Composition 4 |
| | Type | Presence of benzene ring | Kinematic viscosity (25°C) | | | | |
| Case 1 | Dimethicone | No | 1.5 cSt | | C | C | C |
| Case 2 | Dimethicone | No | 6 cSt | | B | B | B |
| Case 3 | Dimethicone | No | 10 cSt | | A | A | A |
| Case 4 | Dimethicone | No | 20 cSt | C | A | A | B |
| Case 5 | Dimethicone | No | 100 cSt | | A | A | C |
| Case 6 | Diphenylsiloxy phenyl trimethicone | Yes | 50 cSt | | A | A | A |
| Case 7 | Diphenyl dimethicone | Yes | 390 cSt | | A | A | A |

As is clear from the results in Table 1-1, in Cases 1 to 5, (i) when a silicone oil was not blended (i.e., Composition 1) or (ii) when a silicone oil having a kinematic viscosity at 25°C of less than 10 cSt was used (i.e., Compositions 2 and 3), crystals of Compound I were observed despite isododecane being blended as a compatibilizing oil, i.e., the solubility of Compound I in these oils was found to be inferior.

In Cases 1, 2, 4, and 5, crystals of Compound I were observed, i.e., the solubility of Compound I in these oils was found to be inferior.

In Cases 3 to 5, when a silicone oil having a kinematic viscosity at 25°C of 10 cSt or more and lacking a benzene ring and isododecane (compatibilizing oil) were used (i.e., Compositions 2 and 3), solubility of Compound I in oil was found to have improved.

In Cases 6 and 7, when a silicone oil having a benzene ring in the structure thereof was used (i.e., Compositions 2 to 4), solubility of Compound I in oil was found to be satisfactory regardless of the presence or absence of blending of a compatibilizing oil. This is presumably because silicone oils having a benzene ring in the structure thereof are generally highly polar and exhibit satisfactory compatibility with polar oils.

### <<Cases 8 to 14 Evaluation of solubility of Compound I in oil>>

Based on the composition in Table 2-1 below, the compositions of Cases 8 to 14 shown in Table 2-2 were prepared. Compound I was added in an amount equal to or greater than the solubility of Compound I with respect to the polar oil used, and the amount of crystal precipitation of Compound I was evaluated in the same manner as above. Evaluation results are shown in Table 2-2.

**[Table 2-1]**

| (Table 2-1 Composition) | | | | |
|---|---|---|---|---|
| | Composition 1 | Composition 2 | Composition 3 | Composition 4 |
| Compound I | 4.5 | 4.5 | 4.5 | 4.5 |
| Isododecane (compatibilizing oil) | 35.5 | 25.5 | 10 | - |
| Diethoxyethyl succinate (polar oil) | 60 | 60 | 60 | 60 |
| Silicone oil | - | 10 | 25.5 | 35.5 |
| Total | 100 | 100 | 100 | 100 |

| | | | | |
|---|---|---|---|---|
| *Numerical values in table represent blending amounts. | | | | |

**[Table 2-2]**

| (Table 2-2 Evaluation of solubility of Compound I) | | | | | | | |
|---|---|---|---|---|---|---|---|
| | Silicone oil | | | Composition 1 | Composition 2 | Composition 3 | Composition 4 |
| | Type | Presence of benzene ring | Kinematic viscosity (25°C) | | | | |
| Case 8 | Dimethicone | No | 1.5 cSt | | C | C | C |
| Case 9 | Dimethicone | No | 6 cSt | | B | B | B |
| Case 10 | Dimethicone | No | 10 cSt | | A | A | A |
| Case 11 | Dimethicone | No | 20 cSt | C | A | A | B |
| Case 12 | Dimethicone | No | 100 cSt | | A | A | C |
| Case 13 | Diphenylsiloxy phenyl trimethicone | Yes | 50 cSt | | A | A | A |
| Case 14 | Diphenyl dimethicone | Yes | 390 cSt | | A | A | A |

As is clear from the results in Table 2-2, the solubility of Compound I in Cases 8 to 14 exhibited almost the same tendencies as in Cases 1 to 7 in Table 1-2, wherein the types of polar oils blended were different.

### <<Cases 15 to 21 Evaluation of solubility of Compound I in oil>>

Based on the composition in Table 3-1 below, the compositions of Cases 15 to 21 shown in Table 3-2 were prepared. Compound I was added in an amount equal to or greater than the solubility of Compound I with respect to the polar oil used, and the amount of crystal precipitation of Compound I was evaluated in the same manner as above. Evaluation results are shown in Table 3-2.

**[Table 3-1]**

| (Table 3-1 Composition) | | | | |
|---|---|---|---|---|
| | Composition 1 | Composition 2 | Composition 3 | Composition 4 |
| Compound I | 3.5 | 3.5 | 3.5 | 3.5 |
| Isododecane (compatibilizing oil) | 36.5 | 26 | 10.5 | - |
| Bis-ethoxydiglycol cyclohexane-1,4-dicarboxylate (polar oil) | 60 | 60 | 60 | 60 |
| Silicone oil | - | 10.5 | 26 | 36.5 |
| Total | 100 | 100 | 100 | 100 |

| | | | | |
|---|---|---|---|---|
| *Numerical values in table represent blending amounts. | | | | |

**[Table 3-2]**

| (Table 3-2 Evaluation of solubility of Compound I) | | | | | | | |
|---|---|---|---|---|---|---|---|
| | Silicone oil | | | Composition 1 | Composition 2 | Composition 3 | Composition 4 |
| | Type | Presence of benzene ring | Kinematic viscosity (25°C) | | | | |
| Case 15 | Dimethicone | No | 1.5 cSt | | C | C | C |
| Case 16 | Dimethicone | No | 6 cSt | | B | B | B |
| Case 17 | Dimethicone | No | 10 cSt | | A | A | A |
| Case 18 | Dimethicone | No | 20 cSt | C | A | A | B |
| Case 19 | Dimethicone | No | 100 cSt | | A | A | C |
| Case 20 | Diphenylsiloxy phenyl trimethicone | Yes | 50 cSt | | A | A | A |
| Case 21 | Diphenyl dimethicone | Yes | 390 cSt | | A | A | A |

As is clear from the results in Table 3-2, the solubility of Compound I in Cases 15 to 21 exhibited almost the same tendencies as in Cases 1 to 7 in Table 1-2 and in Cases 8 to 14 in Table 2-2, wherein the types of polar oils blended were different.

### <<Examples 1 and 2, Comparative Example 1, and Reference Examples 1 and 2>>

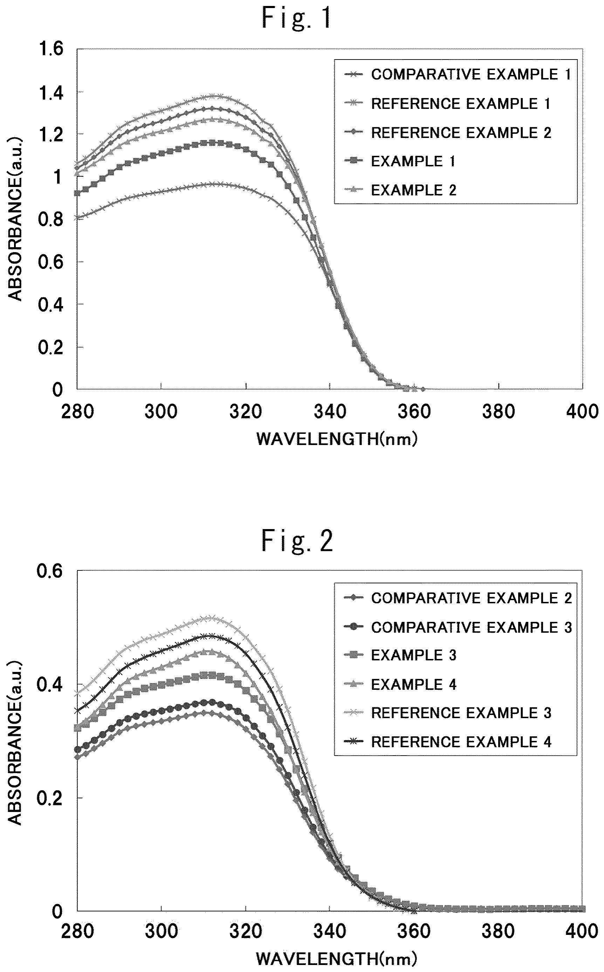

Based on the compositions in Table 4 below, an oil-in-water emulsion cosmetic of each case was prepared. Approximately 50 mg of each cosmetic sample was then dropped onto a hydrophilic surface of a test plate having the hydrophilic surface made of transparent polymethyl methacrylate (PMMA) in a size of 5 cm × 5 cm, spread over the entire surface of the test plate with a finger, and dried to prepare a test sample. Using a spectrophotometer (Model U-3500 auto-recording spectrophotometer, manufactured by Hitachi, Ltd.), light absorbance at 280 to 400 nm was measured. Measurement results are shown in FIG. 1.

**[Table 4]**

| (Table 4) | | | | | | |
|---|---|---|---|---|---|---|
| | | Comparative Example 1 | Reference Example 1 | Reference Example 2 | Example 1 | Example 2 |
| Polar oil | Diisopropyl sebacate | 20 | 20 | 20 | 20 | 20 |
| Compound I | | 3.0 | 3.0 | 3.0 | 3.0 | 3.0 |
| First silicone oil*¹ | Methylpolysiloxane (20 cSt) | - | - | - | 2.5 | - |
| | Methylpolysiloxane (100 cSt) | - | - | - | - | 2.5 |
| Second silicone oil*² (compatibilizing oil) | Decamethyltetrasiloxane silicone (1.5 cSt) | 5 | 2.5 | 2.5 | 2.5 | 2.5 |
| Other silicone oil*³ | Diphenylsiloxy phenyl trimethicone (50 cSt) | - | 2.5 | - | - | - |
| | Diphenyl dimethicone (390 cSt) | - | - | 2.5 | - | - |
| Water | Ion-exchanged water | 56.1 | 56.1 | 56.1 | 56.1 | 56.1 |
| | EDTA-3Na·2H₂O | 0.1 | 0.1 | 0.1 | 0.1 | 0.1 |
| | Glycerin | 3.0 | 3.0 | 3.0 | 3.0 | 3.0 |
| | Dipropylene glycol | 2.0 | 2.0 | 2.0 | 2.0 | 2.0 |
| Additional component | 1,3-butylene glycol | 7.0 | 7.0 | 7.0 | 7.0 | 7.0 |
| | Beheneth-20 | 0.5 | 0.5 | 0.5 | 0.5 | 0.5 |
| | Stearyl alcohol | 0.3 | 0.3 | 0.3 | 0.3 | 0.3 |
| | Behenyl alcohol | 1.0 | 1.0 | 1.0 | 1.0 | 1.0 |
| | Ethanol | 2.0 | 2.0 | 2.0 | 2.0 | 2.0 |
| Total | | 100 | 100 | 100 | 100 | 100 |

| | | | | | | |
|---|---|---|---|---|---|---|
| *Numerical values in table represent blending amounts. *1 "First silicone oil" refers to a silicone oil having a kinematic viscosity at 25°C of 10 cSt or more and lacking a benzene ring. Kinematic viscosity at 25°C is described within parentheses. *2 "Second silicone oil" refers to a silicone oil having a kinematic viscosity at 25°C of 6 cSt or less. Kinematic viscosity at 25°C is described within parentheses. *3 "Third silicone oil" refers to a silicone oil corresponding to neither of the "first silicone oil" and "second silicone oil" above. | | | | | | |

As shown in FIG. 1, Reference Examples 1 and 2 were both found to exhibit high light absorbance as expected. This is presumably because Reference Examples 1 and 2 used silicone oils having a benzene ring in the structures thereof, and thus are highly polar and exhibit satisfactory compatibility with polar oils, resulting in the satisfactory solubility of Compound I.

Examples 1 and 2 both exhibited higher light absorbance, compared to Comparative Example 1. This suggests that the solubility of Compound I in the compositions of Examples 1 and 2 was improved, compared to that of Comparative Example 1.

### <<Examples 3 and 4, Comparative Examples 2 and 3, and Reference Examples 3 and 4>>

Based on the compositions in Table 5 below, a water-in-oil emulsion cosmetic of each case was prepared. Except that a sample of each cosmetic was applied to a hydrophobic surface of a test plate having the hydrophobic surface, light absorbance at 280 to 400 nm was then measured in the same manner as above. Measurement results are shown in FIG. 2.

**[Table 5]**

| (Table 5) | | | | | | | |
|---|---|---|---|---|---|---|---|
| | | Comparative Example 2 | Comparative Example 3 | Example 3 | Example 4 | Reference Example 3 | Reference Example 4 |
| Polar oil | Diisopropyl sebacate | 10 | 10 | 10 | 10 | 10 | 10 |
| Compound I | | 3.0 | 3.0 | 3.0 | 3.0 | 3.0 | 3.0 |
| Hydrocarbon oil (compatibilizing oil) | Isododecane | 10 | 10 | 10 | 10 | 10 | 10 |
| Second silicone oil*² (compatibilizing oil) | Decamethyltetrasiloxane silicone (1.5 cSt) | 10 | 5.0 | 5.0 | 5.0 | 5.0 | 5.0 |
| | Methylpolysiloxane (6 cSt) | 5.0 | 10 | 5.0 | 5.0 | 5.0 | 5.0 |
| First silicone oil *¹ | Methylpolysiloxane (20 cSt) | - | - | 5.0 | - | - | - |
| | Methylpolysiloxane (100 cSt) | - | - | - | 5.0 | - | - |
| Other silicone oil*³ | Diphenylsiloxy phenyl trimethicone (50 cSt) | - | - | - | - | 5.0 | - |
| | Diphenyl dimethicone (390 cSt) | - | - | - | - | - | 5.0 |
| Water | Ion-exchanged water | 52.6 | 52.6 | 52.6 | 52.6 | 52.6 | 52.6 |
| | PEG-9 polydimethylsiloxyethyl dimethicone | 2.5 | 2.5 | 2.5 | 2.5 | 2.5 | 2.5 |
| Additional component | Disteardimonium hectorite | 0.7 | 0.7 | 0.7 | 0.7 | 0.7 | 0.7 |
| | EDTA-3Na | 0.2 | 0.2 | 0.2 | 0.2 | 0.2 | 0.2 |
| | Ethanol | 6.0 | 6.0 | 6.0 | 6.0 | 6.0 | 6.0 |
| Total | | 100 | 100 | 100 | 100 | 100 | 100 |

| | | | | | | | |
|---|---|---|---|---|---|---|---|
| *Numerical values in table represent blending amounts. *1 "First silicone oil" refers to a silicone oil having a kinematic viscosity at 25°C of 10 cSt or more and lacking a benzene ring. Kinematic viscosity at 25°C is described within parentheses. *2 "Second silicone oil" refers to a silicone oil having a kinematic viscosity at 25°C of 6 cSt or less. Kinematic viscosity at 25°C is described within parentheses. *3 "Third silicone oil" refers to a silicone oil corresponding to neither of the "first silicone oil" and "second silicone oil" above. | | | | | | | |

As is clear from the results in FIG. 2, Examples 3 and 4 both exhibited higher light absorbance, compared to Comparative Examples 2 and 3. This suggests that the solubility of Compound I in the compositions of Examples 3 and 4 was improved, compared to that of Comparative Examples 2 and 3.

## Claims

1. A cosmetic composition, comprising
Compound I having a structure of formula (I) below: 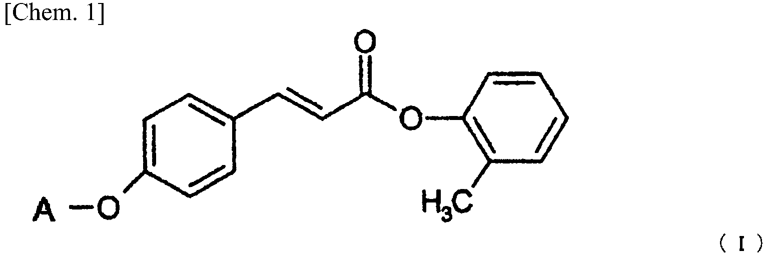
wherein -OA represents an alkoxy group;
a polar oil;
a first silicone oil having a kinematic viscosity at 25°C of 10 cSt or more and lacking a benzene ring; and
a compatibilizing oil compatible with both the polar oil and the silicone oil.

2. The cosmetic composition according to claim 1, wherein the compatibilizing oil is a hydrocarbon oil and/or a second silicone oil having a kinematic viscosity at 25°C of 6 cSt or less.

3. The cosmetic composition according to claim 1, wherein the Compound I is 0.1 to 15 parts by mass relative to 100 parts by mass in total of the Compound I, the polar oil, the first silicone oil, and the compatibilizing oil.

4. The cosmetic composition according to claim 1, wherein the polar oil is 0.1 to 99 parts by mass relative to 100 parts by mass in total of the Compound I and the polar oil.

5. The cosmetic composition according to claim 1, wherein the first silicone oil is 0.1 to 20 parts by mass relative to 100 parts by mass in total of the Compound I, the polar oil, the first silicone oil, and the compatibilizing oil.

6. The cosmetic composition according to claim 1, wherein the compatibilizing oil is 10 to 90 parts by mass relative to 100 parts by mass in total of the first silicone oil and the compatibilizing oil.

7. An oil-in-water emulsion cosmetic, comprising the cosmetic composition according to any one of claims 1 to 6.

8. The oil-in-water emulsion cosmetic according to claim 7, wherein content of the Compound I is 0.01 to 10% by mass.

9. The oil-in-water emulsion cosmetic according to claim 7, wherein content of the polar oil is 0.1 to 50% by mass.

10. A water-in-oil emulsion cosmetic, comprising the cosmetic composition according to any one of claims 1 to 6.

11. The water-in-oil emulsion cosmetic according to claim 10, wherein content of the Compound I is 0.01 to 10% by mass.

12. The water-in-oil emulsion cosmetic according to claim 10, wherein content of the polar oil is 0.1 to 50% by mass.
